# EUROPÄISCHE PATENTANMELDUNG

(11) **EP 0 623 349 A1**
(43) Veröffentlichungstag der Anmeldung: **09.11.1994**
(21) Anmeldenummer: 94106284.6
(22) Anmeldetag: 22.04.1994
(51) Int. Cl.: A61K 31/71, A61K 47/22, A61L 27/00

(54) **Lösungsmittel für ein schwerlösliches Gentamicin-Salz**

(30) Priorität: 05.05.1993 DE 4314871
(71) Anmelder: MERCK PATENT GmbH, D-64293 Darmstadt (DE)
(72) Erfinder: Cimbollek, Monika, D-68167 Mannheim (DE); Nies, Berthold, Dr., D-64407 Fränkisch-Grumbach (DE)

(57) **Zusammenfassung**

Die Erfindung betrifft ein Lösungsmittel für das Salz von Gentamicin mit 3-p-Methoxybenzyliden-6-hydroxy-4'-methoxyflavanon-6-phosphat, das aus einem Gemisch aus 6 bis 9,75 Volumenteilen Tetrahydrofuran und 4 bis 0,25 Volumenteilen Wasser besteht. In diesen Lösungsmitteln ist der Wirkstoff bis zu ca. 50 Gew.% löslich. Eine derartige Lösung eignet sich besonders für die Beladung von pharmazeutischen Trägern mit diesem Wirkstoff.

## Beschreibung

Die Erfindung betrifft ein Lösungsmittel für ein schwerlösliches Salz des pharmazeutischen Wirkstoffes Gentamicin sowie ein Verfahren zur Beladung von pharmazeutischen Wirkstoffträgern mit diesem Salz.

Gentamicin gehört zu den Aminoglycosid-Antibiotika und zeichnet sich durch eine ausgeprägte Breitbandwirkung aus. Aufgrund seines spezifischen Wirkspektrums hat sich Gentamicin insbesondere auch bei der lokalen Behandlung von Knocheninfektionen bewährt. In der Regel kommt Gentamicin als Schwefelsäuresalz zur Anwendung. Dieses Salz ist jedoch in Wasser bzw. physiologischen Medien leicht löslich. Eine lokale Behandlung mit Gentamicin-Sulfat, beispielsweise durch Implantation von mit Gentamicin-Sulfat beladenen Wirkstoffträgern am Infektions- bzw. Behandlungsort ist in der Regel dadurch charakterisiert, daß sich anfänglich kurzfristig eine hohe Wirkstoffkonzentration aufbaut, die dann sehr schnell abfällt. Dies läßt sich etwa durch in-vitro-Elutionsversuche oder durch Messung der Serumkonzentration in-vivo nachweisen. Therapeutisch wünschenswert ist jedoch eine langanhaltende, je nach Beschickungsmenge möglichst über Wochen oder Monate gleichbleibende bzw. nur langsam abnehmende Wirkstoffkonzentration. Aufgrund der guten Wasserlöslichkeit von Gentamicin-Sulfat hat sich eine protrahierte Freisetzung durch entsprechende galenische Formulierungen dieses Wirkstoffes bzw. der Wirkstoffträger nicht realisieren lassen.

Aus DE 34 31 534 ist das Salz von Gentamicin mit 3-p-Methoxybenzyliden-6-hydroxy-4'-methoxyflavanon-6-phosphat, im weiteren als "Gentamicin-Salz" bezeichnet, bekannt. Dieses Salz ist in Wasser und wäßrigen Medien schwer löslich. Es hat sich aber gezeigt, daß aus diesem Salz im wäßrigen Medium langsam Gentamicin abgespalten und freigesetzt wird. Daher ist dieses Gentamicin-Salz als solches bereits als Depot-Antibiotikum einsetzbar bzw. gut geeignet, als antibiotischer Wirkstoff in pharmazeutischen Trägern mit protrahierter Freisetzung eingesetzt zu werden. Entsprechende Befunde sind beispielsweise in Z. Zahnärztl. Implantol. III, 179 bis 182 (1987) wiedergegeben.

Die Schwerlöslichkeit des Gentamicin-Salzes bringt aber bei der Beladung von Wirkstoffträgern erhebliche Nachteile mit sich. Bei einer maximalen Löslichkeit des Gentamicin-Salzes in Wasser von ca. 0,5 Gew.% können poröse Wirkstoffträger wie etwa Calciumphosphatkeramiken oder strukturiertes Kollagen durch Tränken mit entsprechender Lösung und Trocknen naturgemäß nur mit einer begrenzten Menge an Wirkstoff beschickt werden. Eine wiederholte Durchführung des Tränk- und Trocknungsvorganges ist, sofern überhaupt praktikabel, aufwendig und führt darüberhinaus zu ungleichmäßiger Wirkstoffverteilung im Trägermaterial und damit zu erheblichen Streuungen in den Freisetzungsraten. Gleiches gilt für den entsprechenden Einsatz von wäßrigen Suspensionen dieses Wirkstoffs.

Der Erfindung lag daher die Aufgabe zugrunde, ein Lösungsmittel bzw. ein Lösungsmittelsystem aufzufinden, in dem das Gentamicin-Salz gut löslich ist und mit dem Gentamicin-Salzlösungen mit einem Gehalt von mehr als 10 Gew.% herstellbar sind, die sich vorzugsweise für die Behandlung von pharmazeutischen Trägern mit diesem Wirkstoff eignen.

Systematische Untersuchungen haben nun gezeigt, daß das Gentamicin-Salz nicht nur in Wasser, sondern auch in praktisch allen üblichen organischen Lösungsmitteln schwerlöslich ist. Tabelle 1 zeigt die maximalen Löslichkeiten des Gentamicin-Salzes in verschiedenen Lösungsmitteln und Tabelle 2 die in verschiedenen Lösungsmittelgemischen.

**Tabelle 1**

| Maximale Löslichkeiten des Gentamicin-Salzes in verschiedenen Lösungsmitteln | |
|---|---|
| Lösungsmittel | max. Löslichkeit in mg/ml (20 °C) |
| Wasser | 0,6 |
| Wasser pH 9 (NH₄OH) | ≦ 100 |
| Methanol | 9 |
| Ethanol | 0,1 |
| Isopropanol | < 1 |
| Aceton | < 1 |
| Chloroform | 5 |
| Dichlormethan | < 10 |
| 1,1,1-Trichlorethan | < 5 |
| Diisopropylether | < 5 |
| Methyl-t-butylether | < 5 |
| THF | 50 |
| Methylethylketon | < 5 |
| Cyclohexan | < 5 |
| DMF | < 5 |

**Tabelle 2**

| Maximale Löslichkeiten des Gentamicin-Salzes in verschiedenen Lösungsmittelgemischen | |
|---|---|
| Lösungsmittelgemisch (Vol.-Verh.) | max. Löslichkeit in mg/ml (20 °C) |
| Methanol/Dichlormethan 7:3 | 10 |
| Methanol/Essigester 1:1 | < 1 |
| Aceton/Methanol 7:3 | < 1 |
| Isopropanol/Methanol 7:3 | < 1 |
| THF/Methanol 4:1 | 30 (50 °C) |
| DMF/Methanol 1:1 | 50 (50 °C) |
| Zusätze von 10-50 Vol.-% | |
| Wasser zu Methanol | |
| Ethanol und Aceton | < 1 |

Es läßt sich hieraus ablesen, daß sich mit der überwiegenden Zahl der Lösungsmittel bzw. Gemische nur Lösungen mit einem Gehalt von deutlich unter 1 Gew.% herstellen lassen. In einigen wenigen Fällen lassen sich Gehalte zwischen 1 und ca. 5 Gew.% erreichen. Die höchstmögliche Konzentration von ca. 10 Gew.% läßt sich nur in Wasser mit erhöhtem pH-Wert erhalten. In wäßrig-alkalischer Lösung ist der Wirkstoff jedoch instabil und zersetzt sich, auch in damit behandelnden Trägermaterialien. Für den vorgesehenen Anwendungszweck ist diese Möglichkeit somit ungeeignet. Eine grundlegende Verbesserung der Lösungseigenschaften durch Zusatz von Tensiden oder anderen als Lösungsvermittler bekannten Substanzen, wie etwa höhere Alkohole, tritt ebenfalls nicht ein.

Völlig überaschend wurde jedoch nun gefunden, daß Gemische aus 6 bis 9,75 Volumenteilen Tetrahydrofuran und 4 bis 0,25 Volumenteilen Wasser ausgezeichnete Lösungsmittel für das ansonsten schwerlösliche Gentamicin-Salz darstellen. In diesen Gemischen läßt sich das Salz ohne weiteres bis zu Konzentrationen um ca. 50 Gew.% lösen.

Gegenstand der Erfindung ist somit ein Lösungsmittel für das Gentamicin-Salz, das aus einem Gemisch aus 6 bis 9,75 Volumenteilen Tetrahydrofuran und 4 bis 0,25 Volumenteilen Wasser besteht.

Gegenstand der Erfindung ist auch die Verwendung eines derartigen Gemisches als Lösungsmittel für das Gentamicin-Salz sowie ein Verfahren zur Herstellung von Gentamicin-Salzlösungen bei dem man das Salz in einem solchen Gemisch löst.

Gegenstand der Erfindung sind weiterhin Lösungen des Gentamicin-Salzes in derarigen Gemischen sowie ein Verfahren zur Behandlung von pharmazeutischen Wirkstoffträgern mit diesem Salz, bei dem man den Träger mit einer solchen Lösung behandelt.

Tabelle 3 zeigt die maximalen Löslichkeiten des Gentamicin-Salzes in Tetrahydrofuran-Wasser-Gemischen verschiedener Volumenzusammensetzungen.

**Tabelle 3**

| Maximale Löslichkeiten des Gentamicin-Salzes in Tetrahydrofuran-Wasser-Gemischen verschiedener Volumenzusammensetzungen | |
|---|---|
| THF/H₂O-Gemisch (Vol.-Verh.) | max. Löslichkeit in mg/ml (20 °C) |
| 10,00 : 0,00 | 50 |
| 9,75 : 0,25 | 140 |
| 9,5 : 0,5 | 250 |
| 9,0 : 1,0 | 600 |
| 8,0 : 2,0 | 550 |
| 7,0 : 3,0 | 500 |
| 6,0 : 4,0 | 140 |
| 5,0 : 5,0 | < 100 |

Man erkennt bei THF/Wasser-Gemischen im Zusammensetzungsbereich 6 bis 9,75 zu 4 bis 0,25 einen dramatischen Löslichkeitssprung auf Wirkstoffgehalte der Lösung von 14 bis 60 Gew.%. Im Bereich Tetrahydrofuran zu Wasser zwischen 7:3 und 9:1 ist die Löslichkeit maximal. Außerhalb dieser Bereiche liegen die Löslichkeiten unter 10 Gew.%. Dieser Befund ist überraschend und aufgrund der offensichtlich ansonsten generellen Schwerlöslichkeit des Salzes (siehe Tabellen 1 und 2) nicht vorhersehbar.

Die Herstellung der Gentamicin-Salzlösung erfolgt ganz einfach durch Einbringen des Wirkstoffes in das erfindungsgemäße Lösungsmittelgemisch und Auflösen unter Rühren. Die gewünschte Konzentration innerhalb des Löslichkeitsbereiches wird durch entsprechende Abstimmung der Mengen an Gentamicin-Salz und Lösungsmittelgemisch eingestellt. Bevorzugte Gemischzusammensetzung ist 9 Volumenteile Tetrahydrofuran zu 1 Volumenteil Wasser. Es hat sich gezeigt, daß bei diesem Verhältnis der Lösungsvorgang am schnellsten abläuft. Eine gelinde Erwärmung auf bis zu etwa 50 °C kann den Auflösungsvorgang beschleunigen. Eine nachfolgende Feinfiltration ist gegebenenfalls zweckmäßig.

Die erfindungsgemäßen Gentamicin-Salzlösungen eignen sich besonders für die Beladung von pharmazeutischen Trägern mit dem Wirkstoff. Im Gegensatz zu den bisherigen Möglichkeiten, bei denen Gentamicin-Salzlösungen mit einem Gehalt von allerhöchstens 10 Gew.% herstellbar waren, sind mit dem erfindungsgemäßen Lösungsmittelgemisch nun hochkonzentrierte Lösungen mit Gehalten um ca. 50 Gew.% verfügbar. Mit solchen Lösungen sind ohne weiteres trägergebundene Depotformen von Gentamicin mit hoher Dosierung und somit hoher lokaler Wirkstoffkonzentration möglich.

Als Trägermaterialien kommen in erster Linie poröse Materialien in Frage, die die Wirkstofflösung gut aufsaugen können. Bevorzugt sind bioaktive und insbesondere bioresorbierbare Materialien, die vorzugsweise in Form von Implantatformkörpern vorliegen. Vornehmlich dienen diese Implantatformkörper dem Knochenersatz bei der Behandlung bzw. Rekonstitution von unfall- oder krankheitsbedingten Knochendefekten. Die Formkörper können aber auch nur als Depotimplantate zur lokalen Wirkstofffreisetzung bei der Bekämpfung oder Prophylaxe von Infektionen dienen.

Typische poröse Biomaterialien für Implantatformkörper sind Calciumphosphate wie insbesondere Calciumphosphatkeramiken. In der Regel bestehen dieses aus Hydroxylapatit synthetischen oder natürlichen Ursprungs. Bevorzugt sind Knochenkeramiken, die aus natürlichem Knochen durch Entfernung des organischen Anteils und Sintern der Mineralphase zur Keramik gewonnen werden können. Besonders geeignet für die Imprägnation mit Wirkstoff ist Spongiosakeramik aufgrund der naturbedingten hohen Porosität. Weitere Calciumphosphatmaterialien sind Tricalciumphosphat und Tetracalciumphosphat, die sinngemäß eingesetzt werden. Poröse Implantatformkörper aus bioinerten Polymermaterialien wie beispielsweise Polytetrafluorethylen ("Teflon") oder aus Biopolymeren wie Kollagen, Gelatine, Polylactiden oder Polyglycoliden können ebenfalls mit der erfindungsgemäßen Gentamicin-Salzlösung behandelt werden. Vorzugsweise liegen diese Materialien in Form von saugfähigen Gebilden mit schwammartiger, vliesartiger oder Gewebestruktur vor. Poröse Verbundmaterialien aus Biokeramiken und Biopolymeren eignen sich ebenfalls als Wirkstoffträger.

Die Beladung der porösen Wirkstoffträger mit der erfindungsgemäßen Gentamicin-Salzlösung erfolgt in der Weise, daß man den Träger mit der Wirkstofflösung durch vollständiges Eintauchen oder Betropfen behandelt bis das aufnahmefähige Volumen vollständig mit der Lösung gefüllt ist. Danach wird der Träger getrocknet, vorzugsweise in einem warmen Luftstrom. Die Trocknung erfolgt relativ rasch, da das Tetrahydrofuran, das den Hauptanteil des Lösungsmittelgemisches ausmacht, schnell verdunstet. Hierdurch erhalten die inneren und äußeren Oberflächen der Formkörper eine weitestgehend gleichmäßige Beschichtung mit dem Wirkstoff. Nach einer gegebenenfalls erforderlichen Sterilisation und Sterilverpakkung sind die mit dem Wirkstoff beladenen Implantatformkörper gebrauchsfertig.

Durch eine sinngemäße Behandlung mit der erfindungsgemäßen Gentamicin-Salzlösung können auch unporöse oder nur oberflächenrauhe Implantate mit einer Wirkstoffschicht überzogen werden. Eine derartige antibiotische Beschichtung mit protrahierter Wirkstofffreisetzung kann vorteilhaft für viele Arten von Endoprothesen aus Metall, Keramik oder Kunststoff sein. Neben der hohen Wirkstoffkonzentration der erfindungsgemäßen Lösung zeigt diese hierbei noch den Vorteil, daß sie Materialien gut benetzt und gleichmäßig überzieht, die von rein wäßrigen Lösungen sonst nicht benetzt werden. Zweckmäßig ist beispielsweise die antibiotische Beschichtung des Femurschaftes von Hüftendoprothesen.

### Beispiel 1: Herstellung der Gentamicin-Salzlösung

In ein Gemisch aus 9 ml Tetrahydrofuran und 1 ml Wasser werden 1,5 g des Salzes von Gentamicin mit 3-p-Methoxybenzyliden-6-hydroxy-4'-methoxyflavanon-6-phosphat gegeben und bei 25 °C und unter Rühren aufgelöst.

### Beispiel 2: Behandlung von Trägern aus Spongiosakeramik

Blöcke von aus natürlicher Spongiosa hergestellten porösen Hydroxylapatitkeramiken der Abmessungen 12,5 x 12,5 x 10 mm werden durch Eintauchen mit der Lösung nach Beispiel 1 getränkt. Die mit Lösung vollgesogenen Keramikblöcke werden entnommen und innerhalb von ca. 2 Stunden im warmen Luftstrom getrocknet. Die gebrauchsfertigen Blöcke enthalten 30 mg Gentamicin-Salz.

### Beispiel 3: Beladung eines Teflonvlieses

Ein kreisförmiges Teflonvlies mit Durchmesser 0,5 cm und Höhe 1,45 mm wird in die Lösung nach Beispiel 1 eingetaucht bis es sich vollständig vollgesogen hat. Danach wird es entnommen und ca. 30 Minuten im warmen Luftstrom getrocknet. Das gebrauchsfertige Vlies enthält 63 mg Gentamicin-Salz.

## Patentansprüche

1. Lösungsmittel für das Salz Von Gentamicin mit 3-p-Methoxybenzyliden-6-hydroxy-4'-methoxyflavanon-6-phosphat, dadurch gekennzeichnet, daß es sich um ein Gemisch aus 6 bis 9,75 Volumenteilen Tetrahydrofuran und 4 bis 0,25 Volumenteilen Wasser handelt.

2. Lösungsmittel nach Anspruch 1, dadurch gekennzeichnet, daß es sich um ein Gemisch aus 7 bis 9 Volumenteilen Tetrahydrofuran und 3 bis 1 Volumenteilen Wasser handelt.

3. Verwendung eines Gemisches aus 6 bis 9,75 Volumenteilen Tetrahydrofuran und 4 bis 0,25 Volumenteilen Wasser als Lösungsmittel für das Salz von Gentamicin mit 3-p-Methoxybenzyliden-6-hydroxy-4'-methoxyflavanon-6-phosphat.

4. Verfahren zur Herstellung einer Lösung des Salzes von Gentamicin mit 3-p-Methoxybenzyliden-6-hydroxy-4'-methoxyflavanon-6-phosphat, wobei diese einen Gehalt von mindestens 10 Gew.% aufweist, dadurch gekennzeichnet, daß man das Salz in einem Gemisch aus 6 bis 9,75 Volumenteilen Tetrahydrofuran und 4 bis 0,25 Volumenteilen Wasser löst.

5. Lösung des Salzes von Gentamicin mit 3-p-Methoxybenzyliden-6-hydroxy-4'-methoxyflavanon-6-phosphat, wobei diese einen Gehalt von mindestens 10 Gew.% aufweist, dadurch gekennzeichnet, daß das Lösungsmittel ein Gemisch aus 6 bis 9,75 Volumenteilen Tetrahydrofuran und 4 bis 0,25 Volumenteilen Wasser ist.

6. Verfahren zur Beladung von pharmazeutischen Wirkstoffträgern mit dem Salz von Gentamicin mit 3-p-Methoxybenzyliden-6-hydroxy-4'-methoxyflavanon-6-phosphat, dadurch gekennzeichnet, daß man den Wirkstoffträger mit einer Lösung nach Anspruch 5 behandelt und dann das Lösungsmittel durch Trocknung entfernt.
